# EUROPEAN PATENT SPECIFICATION

(11) **EP 3 482 777 B1**
(45) Date of publication and mention of the grant of the patent: **16.08.2023**
(21) Application number: 18205220.9
(22) Date of filing: 08.11.2018
(51) Int. Cl.: A61L 2/22, A61L 2/24, B65G 61/00, B08B 9/08, B65D 19/38, B08B 3/02

(54) **DEVICE AND PROCESS FOR CLEANING FLAT OBJECTS**
VORRICHTUNG UND VERFAHREN ZUR REINIGUNG FLACHER GEGENSTÄNDE
APPAREIL ET METHODE POUR NETTOYER DES OBJETS PLATS

(30) Priority: 08.11.2017 IT 201700127049
(43) Date of publication of application: 15.05.2019
(73) Proprietor: Colussi Ermes SRL, 33072 Casarsa Della Delizia (PN) (IT)
(72) Inventor: COLUSSI, Giovanni Battista, 33072 CASARSA DELLA DELIZIA (PN) (IT)
(74) Representative: Bonatto, Marco

(56) References cited:
- EP-A1- 1 243 534
- WO-A1-01/62643
- DE-C1- 4 103 538
- US-A1- 2011 203 622
- US-A1- 2013 112 229

## Description

### Field of the invention

The present invention concerns a device and process able to be used for example to wash, sanitize or sterilize substantially flat objects like for example pallets, platforms, pans, boxes or plates.

### State of the art

In order to quickly wash items like pallets, crates and boxes made of plastic, metal pans, plates or other items having an overall flat shape used in large industrial or logistical factories like for example large supermarkets, packaging centres and food industries in general it is currently known to use industrial washing machines provided with a washing chamber in which one or more of these items to be washed can be introduced simultaneously, depending on the type of machine.

The washer carries out automatically a quick washing cycle with water and/or a sanitization cycle with steam or chemical disinfectants after which, again depending on the type of machine, it can dry the products with a flow of hot air, centrifuging them or simply leaving them to drip dry.

A drawback of industrial washers currently used is that they do not allow two or more pallets, pans or other flat items stacked together to be washed simultaneously giving a proper and effective clean to the whole surface of the items, in particular also their lower areas or in any case those resting on the other items of the stack.

Therefore, to deal with this currently the items are washed in the industrial washer one at a time.

It is also known to introduce the items to be washed in mobile frames or to hang them on said frames, not stacked, and then wash the racks or mobile frames together with the items in the industrial washer, all together.

However, the author of the present invention deems that pallets, crates or pans loaded on the racks/mobile frames or hung on them not stacked are in any case very bulky and make inefficient use of the space inside the washer. The documents US2013/0112229A1 and US2011/0203622A1 disclose a washing apparatus for a stack of pallets comprising a lifting mechanism configured to tilt the pallets and spraying nozzles. DE4103538A1 discloses an apparatus and method for washing transport boxes,in which four of said transport boxes are loaded on a support frame but are neither being stacked, nor tilted.

US2008295353 discloses a washing and drying apparatus for stacked containers comprising washing nozzles and a rotor so as to dry the containers but does not disclose a lifting system configured to tilt the containers.

A purpose of the present invention is to avoid the aforementioned drawbacks and in particular to provide a washer and a process for washing relatively flat items like for example pallets, boxes and crates made of plastic, metal pans or plates at industrial production rates, with a higher production rate than known washers - in particular than those which wash one item at a time - or better exploiting the space inside the washer or even better washing the whole surface of the items to be washed.

### Summary of the invention

In a first aspect of the present invention such a purpose is achieved with a processing device having the features according to claim **1.**

In a particular embodiment of such a processing device **(1, 1'),** each lifting column comprises a tension member or pusher **(19')** to which three or more sliders **(17, 17')** are fixed so as to be able to slide along the tension member or pusher **(19')** itself and pass from a first condition in which such sliders **(17, 17')** are closer one to another, to a second condition where such sliders **(17, 17')** are more distanced apart one from another.

In a particular embodiment of such a processing device **(1, 1')**, all of the sliders **(17, 17')** of its lifting column are fixed to such a tension member or pusher **(19').**

In a particular embodiment of such a processing device **(1, 1')**, the tension member or pusher **(19')** comprises a beam or small plate that extends along the guide upright **(15, 15')** and can slide along such a upright.

In a particular embodiment of such a processing device **(1, 1')**, a plurality of slots **(192)** are formed in the tension member or pusher **(19'),** one or more sliders **(17, 17')** being able to slide along said slots and also sliding along the guide upright **(15, 15').**

In a second aspect of the invention such a purpose is achieved with a process for processing substantially flat items to be processed, like for example pallets, platforms, pans, crates or plates, having the features according to claim **11.**

Further features of the invention are the object of the dependent claims.

The advantages able to be obtained with the present invention will become clearer, to those skilled in the art, from the following detailed description of some particular non-limiting embodiments, illustrated with reference to the following schematic figures.

### List of Figures

Figure **1** shows a perspective view of a system for washing and moving pallets stacked according to a first and a second embodiment of the invention;
Figure **2** shows a perspective view of the washer of the washing and moving system of Figure **1****;**
Figure **3** shows a washing container of the washer of

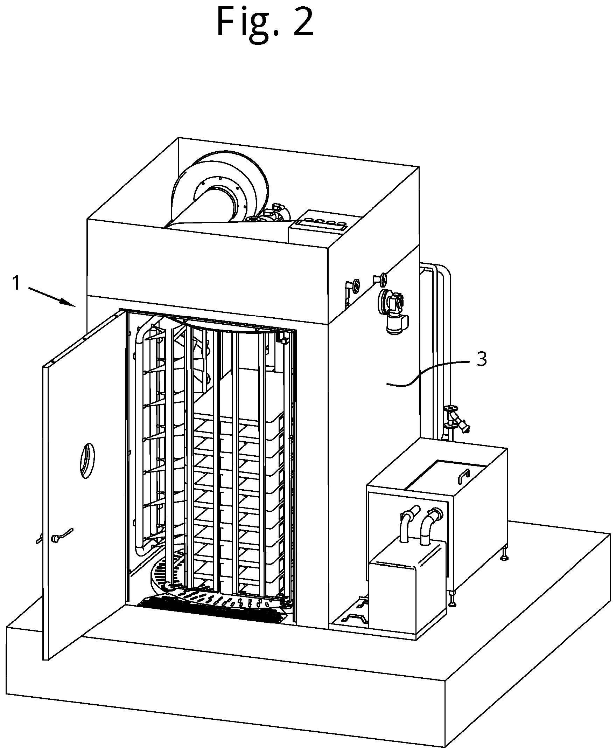

Figure **2****,** according to a first embodiment of the invention, with the pallets loaded, stacked and completely resting on one another;

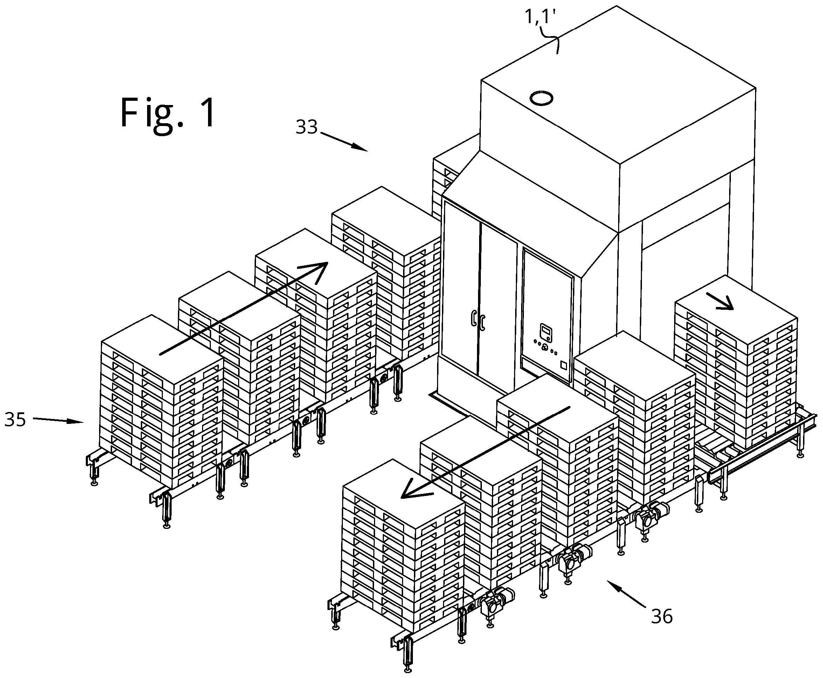

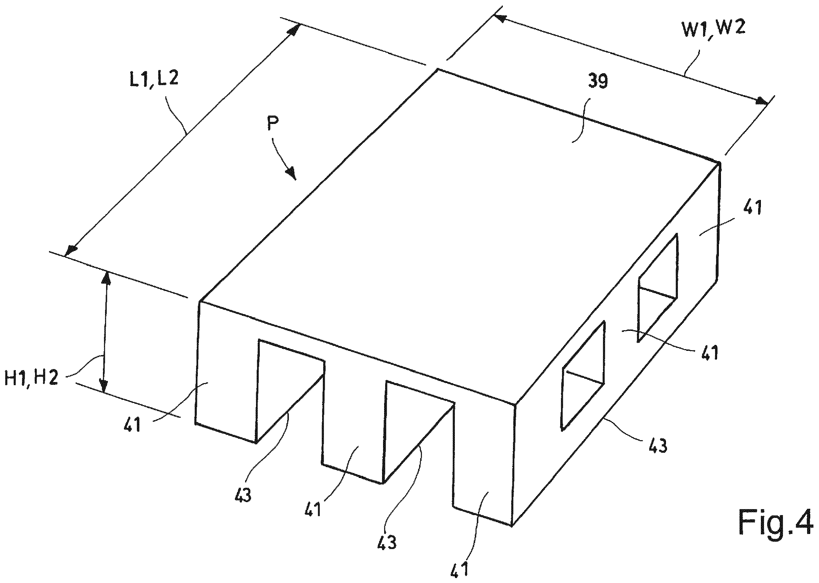

Figure **4** shows a perspective view of an example of a pallet that can be processed in the system of Figure **1****;**

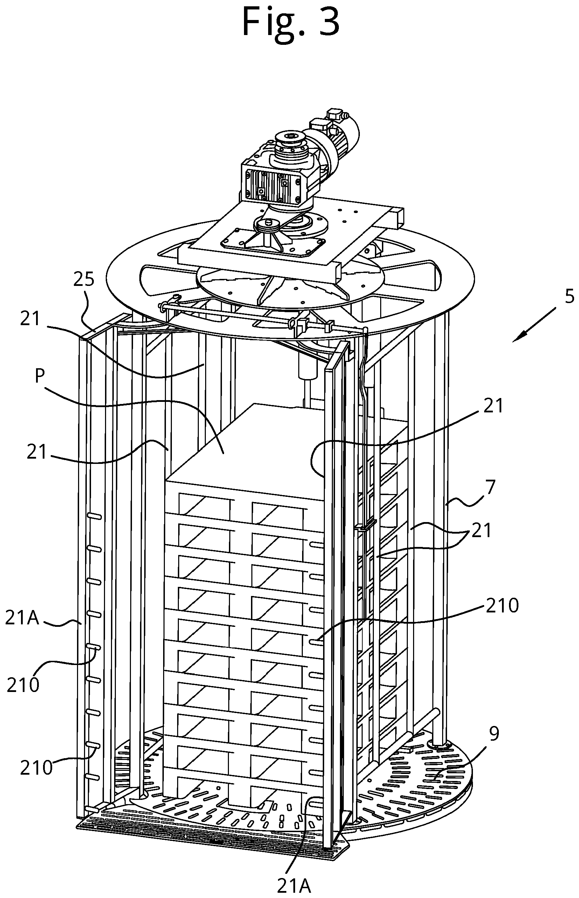

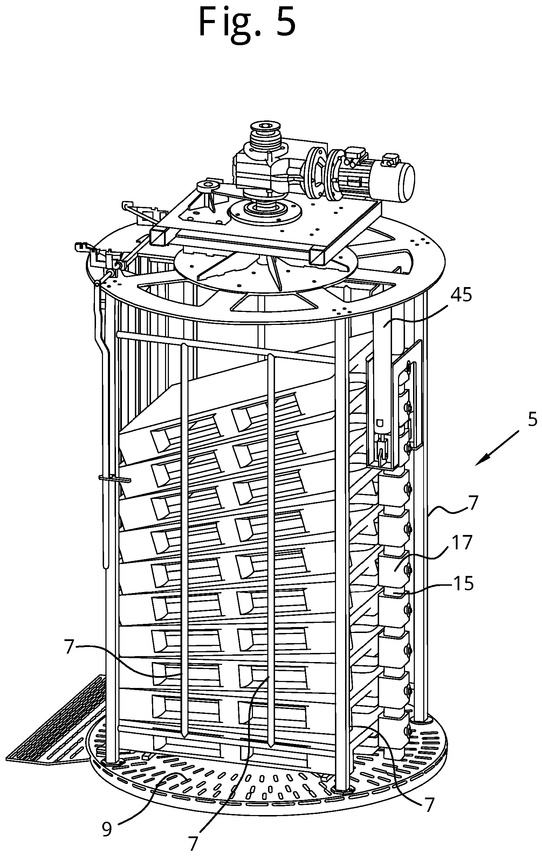

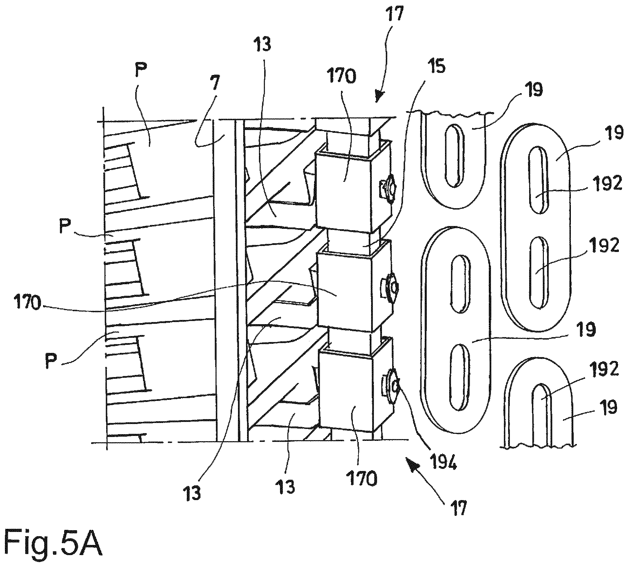

Figure **5** shows the washing container of Figure **3****,** with the pallets loaded, stacked and tilted on one side;
Figure 5A shows a partially exploded view of a detail of the guide upright and of the sliders of the washing container of Figures **3** and **5****,** with the sliders lifted;

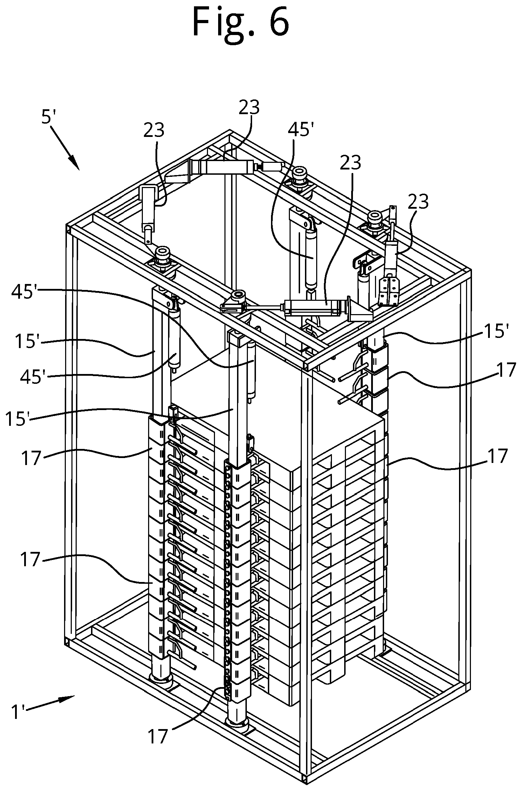

Figure **6** shows a washing container of the washer of
Figure **2****,** according to a second embodiment of the invention, with the pallets loaded, stacked and completely resting on one another;

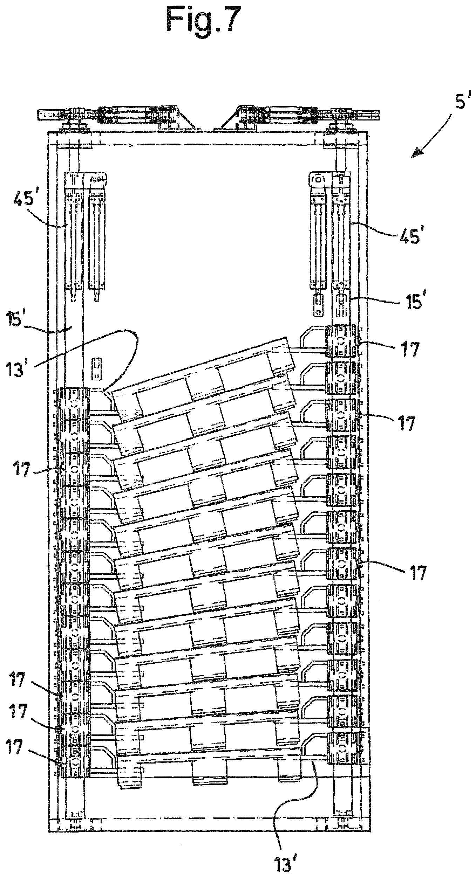

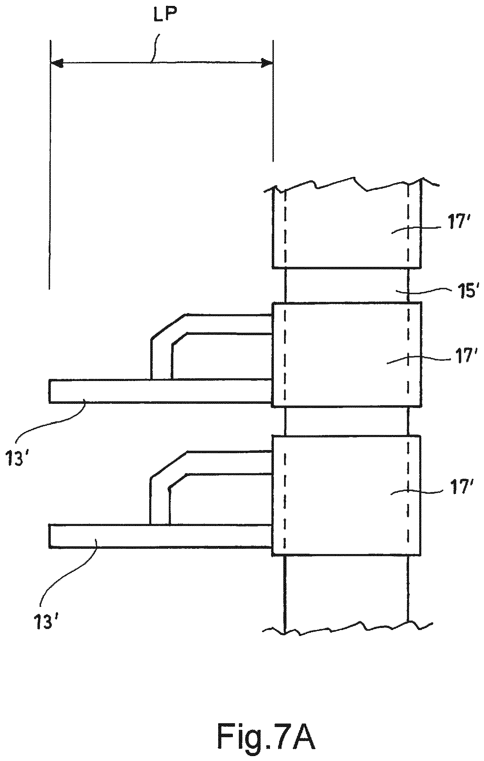

Figure **7** shows a side view of the washing container of
Figure **6****,** with the pallets loaded, stacked and tilted on one side;
Figure **7A** shows an enlarged detail of the view of Figure **7****;**

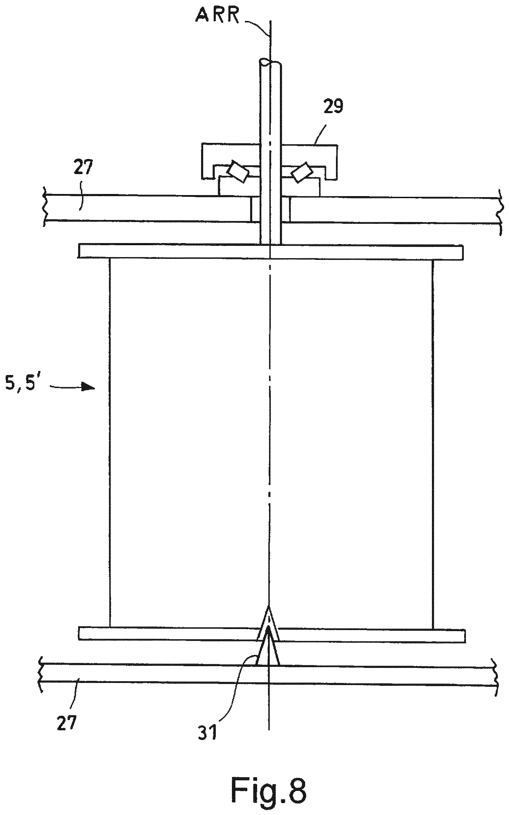

Figure **8** shows a side view of the rotor of the washer of Figure **1****,** according to a first embodiment of the invention;

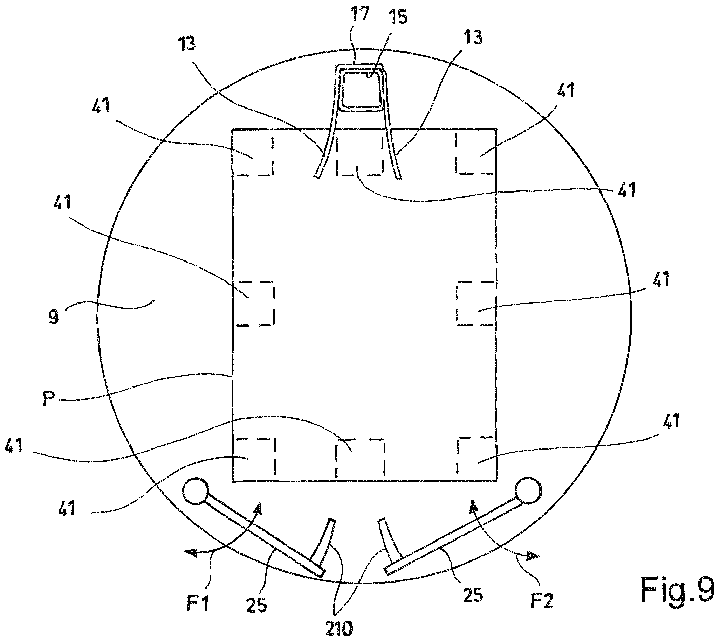

Figure **9** shows a view from above, partially in section, of the washing container of Figure **3****;**

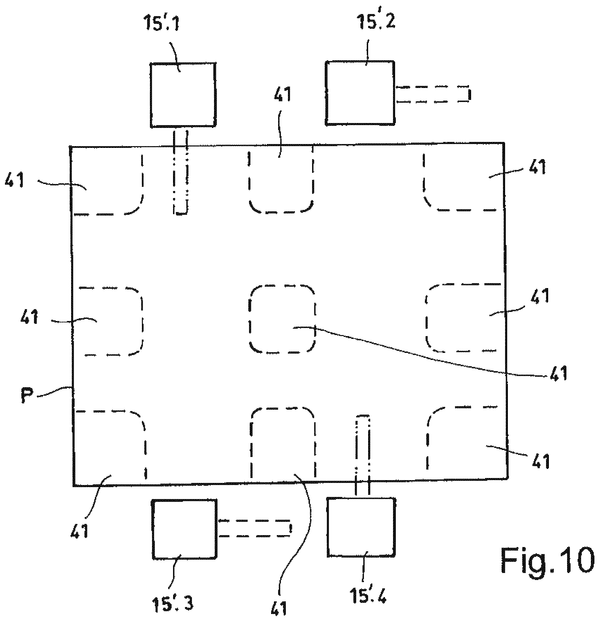

Figure **10** shows a view from above, partially in section, of a pallet stacked and completely resting on the pallets beneath in the washing container of Figure **6****,** and of the relative guide uprights;

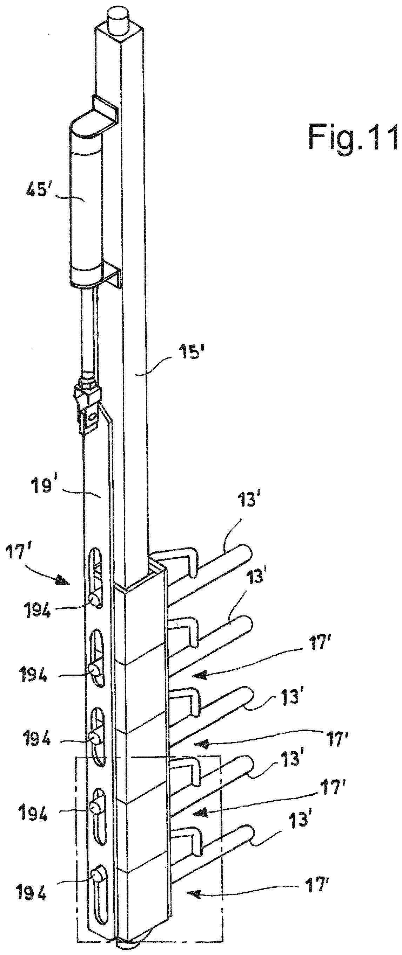

Figure **11** shows a perspective view of a lifting upright of a washer, sanitizer or sterilizer according to a third embodiment of the invention;

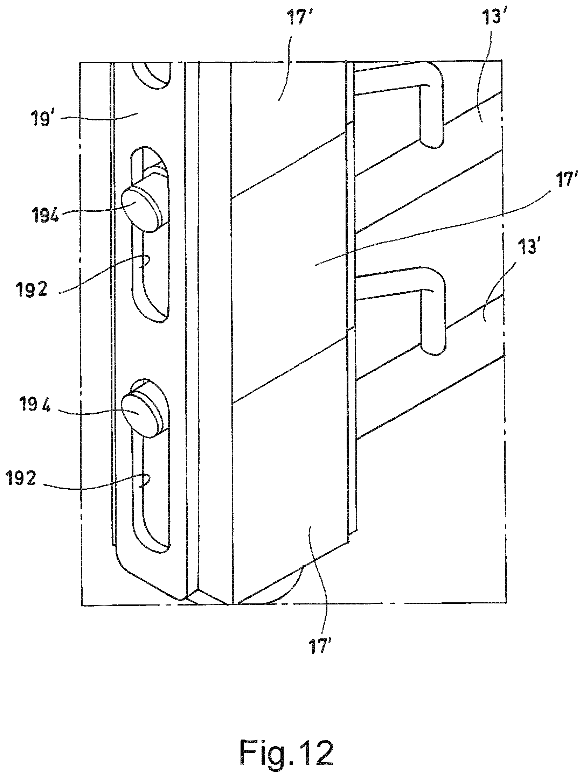

Figure **12** shows a detail of the view of Figure **11****;**

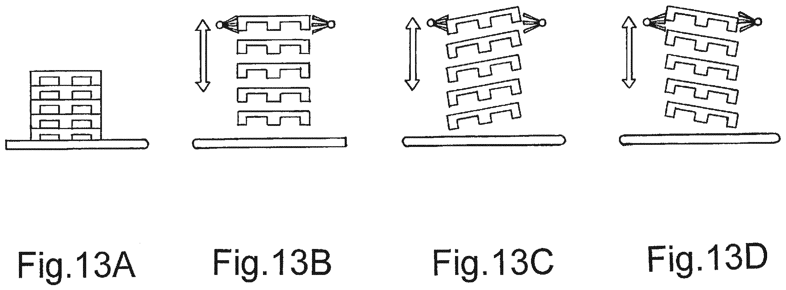

Figures **13A-13F** show some moments of a possible lifting sequence of the washer of Figure **6****;**

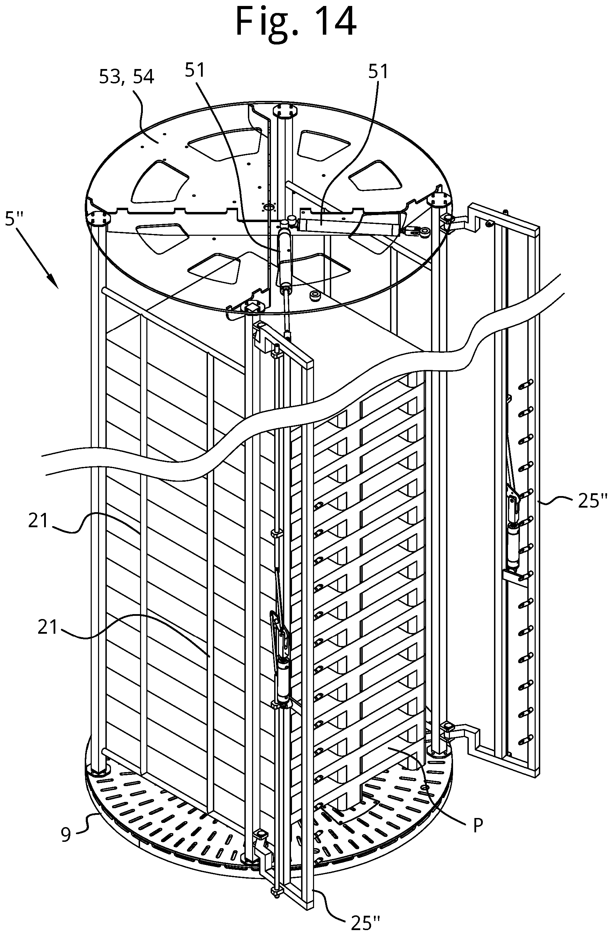

Figure **14** shows a perspective view of a processing container of an automatic washer according to a third embodiment of the present invention, with the lid shown transparent and the leaves of the doors open;

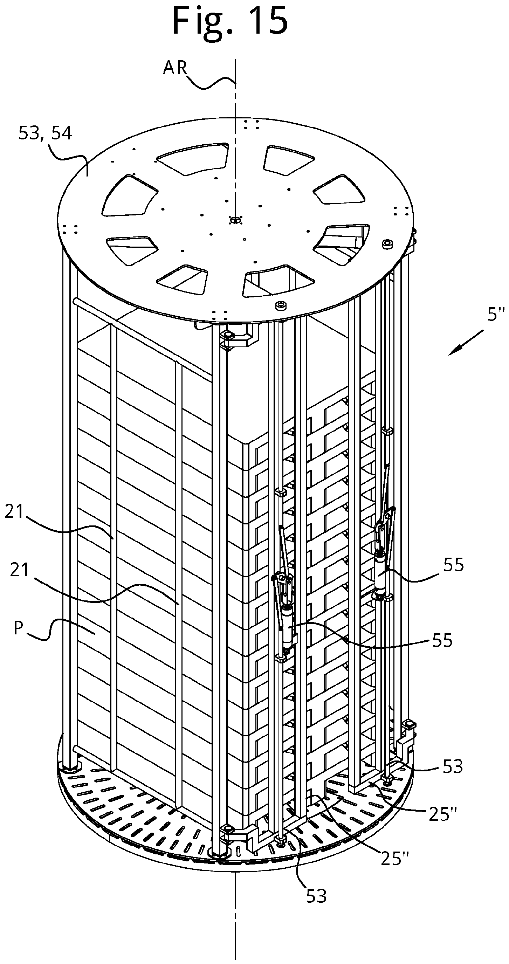

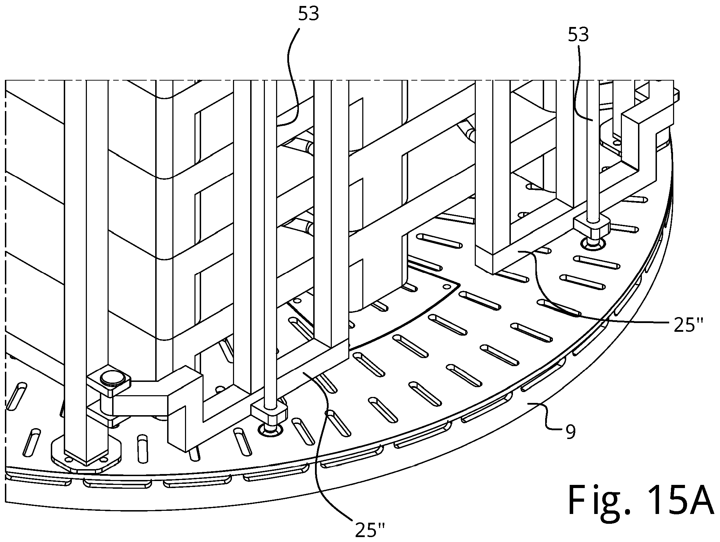

Figure **15** shows a perspective view of the processing container of Figure **14****,** with the doors closed;
Figure 15A shows a perspective view of the lower latch, in locked position, of the processing container of Figure **14****;**

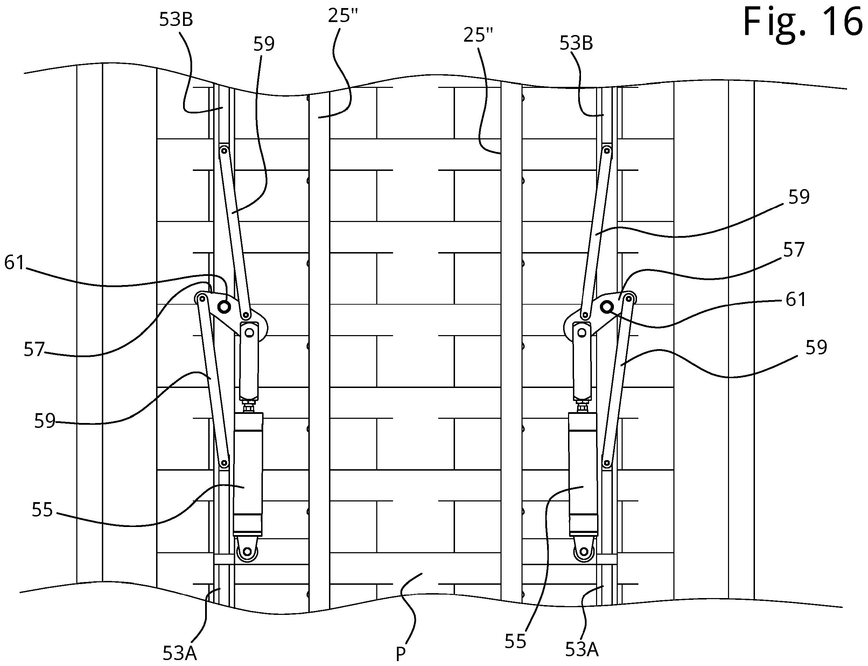

Figure **16** shows a front view of the automatic door-locking system, in unlocked position, of the processing container of Figure **14****;**

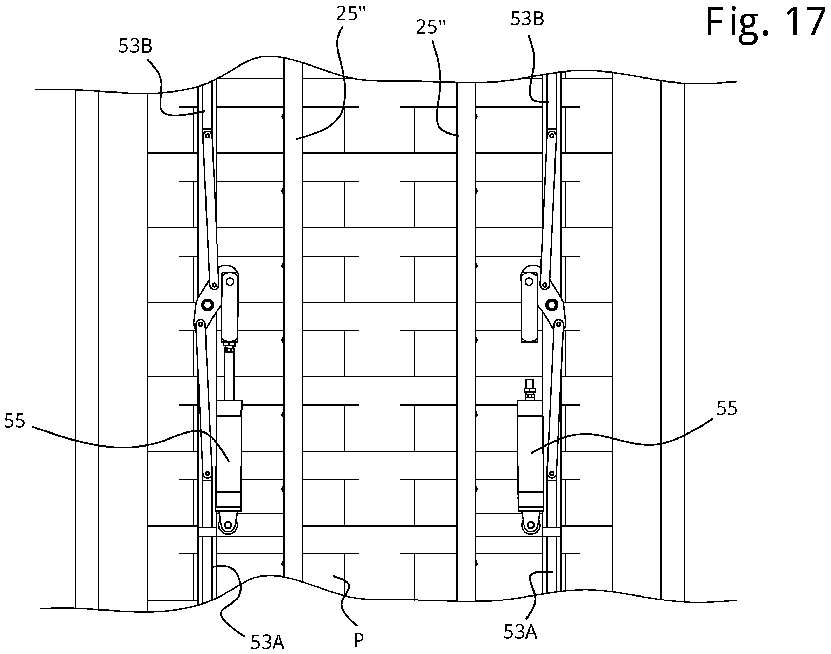

Figure **17** shows a front view of the automatic door-locking system, in unlocked position, of the processing container of Figure **14****.**

### Detailed description

In the present description, unless otherwise specified the expressions "vertical" and "horizontal" are meant to refer to a device, tool or item considered in its normal conditions of use or operation.

Unless otherwise specified the expression "vertical" indicates an inclination comprised between **0-45°** with respect to the acceleration of local gravity.

The attached Figures relate to an automatic washer and to the relative washing and moving system according to some particular embodiments of the invention.

The washer according to the first embodiment is indicated with overall reference numeral **1,** whereas the washer according to the second embodiment is indicated with overall reference numeral **1'**.

Like in Figures **2****,** **7** the automatic washer **1** can comprise a fixed bearing frame **27** possibly enclosed in a metal casing **3** and a washing container or more generally a processing container **5** that forms a chamber or other processing space inside it, arranged to contain a plurality of pallets P or other substantially flat items to be washed, stacked one on top of the other, like for example crates and boxes that may or may not be made of plastic, metal pans, plates.

In the present description, the term substantially flat item unless otherwise specified is meant to indicate an item having a height H**1**, H**2** equal to or less than three quarter or half the shorter out of the width W**1**, W**2** and the length L**1**, L**2** of its base or in any case of its plan (Figure **4**).

The pallet P can for example be a so-called europallet made of plastic or metal - for example aluminium or steel - and having a height H**1**, H**2** for example equal to about **10-20** decimetres or comprised between **13-16** decimetres, a width W**1**, W**2** for example roughly comprised between **0.8-1.2** metres or **0.7-1** metre and a length L**1**, L**2** for example comprised between **1-1.5** metres or **1-1.2** metres.

The pallet P can have a total weight, when dry, for example equal to or greater than **5** kilograms, or equal to or greater than **10** kilograms, equal to or greater than **17** kilograms, equal to or greater than **20-25** kilograms.

Moreover, in the present description the term substantially flat item is meant to indicate not only items that are flat overall but also items that at not flat but thin overall, for example a shell with double curvature, convex on one face and concave on the other.

The pallet P can form an upper resting plane **37,** a plurality of feet **41** that support it and a plurality of strips **43** that join the feet **41** at the bottom.

In so-called europallets there are nine feet **41** connected in groups of three by three strips **43.**

The automatic washer **1** can for example be an industrial washer able to be used for example in large industrial or logistical factories like for example large supermarkets, production or packaging centres of fruit and vegetable products, meats, fish, confectionery, baked products and other foods for people or animals, food industries in general.

As shown in Figures **3****,** **5****,** the processing container **5** can for example have the shape of a cage or metal frame capable of enclosing a stack of pallets or other substantially flat items to be washed, and comprising for example a plurality of uprights **7** and a base **9.**

According to an aspect of the present invention, the washer **1** is also provided with a lifting system **11** arranged for lifting at least part of the flat items P inside the processing container **5** tilting them.

Like in the embodiment of Figures **5****,** **7****,** the lifting system **11** is arranged for lifting at least part of the flat items P inside the processing container **5** tilting them all on a same side.

Like in the embodiments of Figures **2-9****,** the lifting system **11** can comprise a plurality of brackets or lifting hooks **13, 13'** each arranged to rest against a surface of the pallet facing downwards or other hollows or recesses of the pallet P or other item to be washed or more generally processed and lift it only on one side, tilting it.

The brackets or lifting hooks **13** can form forks (Figures 5A, 9).

Each bracket **13, 13'** can comprise for example a bar or pin projecting in a horizontal or in any case non-vertical direction towards the inside of the processing chamber.

Preferably, each bracket **13'** projects in a horizontal or in any case non-vertical direction towards the inside of the processing chamber for a length LP preferably comprised between **20-30** centimetres, and more preferably comprised between **24-27** centimetres (Figure 7A).

The lifting system **11** can comprise at least one guide upright **15** and a plurality of sliders **17** arranged to slide along the upright **15** (Figures 5, 5A), and on each of which a respective bracket or lifting hook is fixed, for example integral with it.

The assembly of a guide upright **15** and of the slider or sliders **17** that slide along it in the present description is for the sake of brevity called "lifting column".

Each guide upright **15** can be made for example as a simple tubular bar with square or rectangular section.

Each slider **17** can comprise for example a sliding sleeve **170** having a more or less tubular shape, for example with a square or rectangular cross section (Figures 5, 5A) slotted on a respective guide upright **15** so as to be able to slide along it.

Each bracket or lifting hook **13** can be fixed on a respective sleeve **170.**

Each slider **17** can slide along the respective guide upright **15** for example sliding as a pad or sliding on wheels or rollers like a carriage.

Advantageously, the sliders **17** mounted on the same guide upright **15** are connected together so as to substantially form a chain or complex rope, and for this purpose they can be connected for example through chain links **19,** connecting rods, tie rods or pieces of cables made of metal or synthetic material: such connections offer, amongst other things, the advantage of separating each slider **17** from the adjacent ones with a constant or in any case very precise distance when the chain of the sliders is stretched out, consequently allowing the pallets P to be separated from one another by very precise distances when they are tilted.

Like in the embodiment of Figure 5A each chain link **19** can form one or more inner slots **192** and in the slot or in the slots **192** of each link **19** the fastening pins **194** of two adjacent sliders **17** are inserted.

Like in the embodiment of Figures **10****,** **11** each lifting column can be provided with a single bar or tie rod **19'** that connects all of the sliders **17'** of that lifting column.

Like in Figure **10** the tie rod **19'** can be shaped like a small plate.

In each tie rod **19'** a plurality of slots **192** can be made in which the fastening pins **194** of the various sliders **17** are inserted - all of the sliders **17** of a same lifting column like in the embodiment of Figure **10****,** or only of part of the sliders **17** of a same lifting column like in embodiments that are not shown.

Such a chain or complex rope, or in any case the assembly of the sliders **17** connected to one another can be actuated by a pneumatic, hydraulic cylinder, or other actuator **45, 45'** for example so as to lift them and lower them, making them slide on the respective guide upright **15, 15'** (Figure **5****,** **7**).

Advantageously, the processing container **5** also comprises one or more containment side bars **21, 21**A against which the pallets P or other items to be washed can rest laterally while they are lifted and tilted, as will be explained more hereinafter.

Advantageously, the processing container **5** comprises a plurality of containment side bars **21, 21**A**,** for example made as vertical uprights, which surround the stack of pallets or other items to be washed containing it laterally and preventing it from coming apart when the pallets P or other items are lifted, centrifuged or more generally during washing.

The assembly of the containment side bars **21, 21**A**,** for example made as vertical uprights forms an example of the aforementioned cage structure.

Like in the embodiments of Figures **3****,** **5** advantageously the containment side bars **21**A located on the side of the stack of items to be washed opposite to a guide upright **15** and/or to a chain of sliders **17** are provided with a plurality of locking brackets **210,** each of which can be made for example as a simple stake (Figure **3**).

As will also be explained hereinafter, each locking bracket **210** has the function of resting against an edge of a pallet P or other item to be washed preventing the edge itself from lifting.

Two rows of locking brackets **210** can for example be advantageously fixed on the two leaves **25** of a door or gate through which the stack of pallets P is introduced in the processing container or extracted from it, so that, by closing, the leaves introduce the locking brackets in as many undercuts or other recesses of the stack of pallets P preventing them from lifting on that side of the stack.

Like in the embodiment of Figures **1-5**A**,** the guide uprights **15,** the lifting hooks or brackets **13,** the container **5** or more generally the lifting system **11** can be mounted on a rotor, or can themselves constitute a rotor, so as to be able to rotate on itself and accelerate the drying of the pallets P or other washed items by centrifuging them, for example at speeds of up to **70** rpm, **80** rpm or **130-140** rpm or comprised between **70-140** rpm.

More in particular, the automatic washer **1** is advantageously arranged to make the rotor turn on itself with a speed equal to or greater than **70** rpm, more preferably equal to or greater than **80** rpm, more preferably equal to or greater than **130-140** rpm, more preferably equal to or greater than **200** rpm, even more preferably equal to or greater than **300** rpm.

Preferably, such a rotor with the pallets P housed in the relative processing container **5, 5', 5"** is centrifuged making it rotate on itself for more complete revolutions, for example for at least **10** revolutions and/or at least for **15** seconds and more preferably for at least **30** seconds.

Preferably, such a rotor is arranged to rotate around a substantially vertical rotation axis, making it easier to load and keep the pallets P or other items to be washed stacked during washing and drying.

In the embodiment of Figures **6****,** **7****,** **9** and in others not shown, however, the guide uprights **15'**, the lifting hooks or brackets **13'**, the container **5'** or more generally the lifting system **11'** can be mounted fixed with respect to the floor on which the washer **1** rests, without the possibility of rotating during washing and drying.

Like for example in the embodiment of Figures **6****,** **7** the lifting system **11'** can comprise at least one pair, and more preferably at least two pairs, of guide uprights **15'** arranged to grip and lift the pallets P or other items to be processed, gripping them with the respective lifting hooks or brackets **13'** on two opposite sides of the stack formed by such pallets P or other items to be processed.

Advantageously, a first pair of guide uprights **15'** is arranged to grip and lift, tilting on one side, the pallets P or other flat items to be washed the plan of which has a first width L**1** or height H**1**, whereas a second pair of guide uprights **15'** is arranged to grip and lift, tilting on one side, pallets P or other flat items to be washed the plan of which has a second and different width L**2** or height H**2**.

For this purpose, each guide upright **15'** can rotate on itself around its own longitudinal axis (Figures **6****,** **10**).

More generally, again for this purpose each guide upright **15'** can reversibly switch, for example by rotating or rotating and translating, from an operating position in which it can grip and lift the pallets (for example like the brackets drawn with dashed lines in Figure **10**), to a non-operating position in which it is not able to grip and lift the pallets (for example like the brackets drawn with a double-dotted and dashed line in Figure **10**).

The lifting system **11'** can for this purpose be provided with one or more actuators **23** - for example pneumatic cylinders - which move the guide uprights **15'** from the operating position to the non-operating position and vice-versa.

The lifting system **11'** can be provided with a detection system of the height H**1**, H**2** of the pallets P or of the other substantially flat items to be processed, so as to be able to automatically control the switching of the guide uprights from the operating position to the non-operating position and vice-versa.

Such a detection system of the height can comprise a simple photocell or other bulk detector that detects the height of the stack: from it, knowing the number of pallets P of the stack, it is possible to obtain the height H**1**, H**2** of the single pallets P or other items to be washed.

Moreover, again for this purpose and in the case in which the pallets P are europallets or in any case substantially flat items with a substantially square or rectangular shape in plan, two guide uprights **15'** are arranged to rest, hook or in any case grip the pallets P at two opposite sides of the stack of pallets P and along or close to a diagonal of the plan of the pallets (Figure **10**).

Advantageously, the rotor to which the processing container **5** belongs is hung at the fixed frame **27** of the washer **1, 1'** through a suitable bearing **29,** like for example a thrust bearing (Figure **8**).

Advantageously, the bearing **29** does not constrain the inclination of the rotation axis ARR of the processing container **5** with respect to the vertical in a particularly rigid manner.

Advantageously, the vertical alignment of the rotation axis ARR is constrained and made particularly precise through a centring pin **31,** coaxial with the bearing **29** and having for example a conical or in any case tapered shape.

The centring pin **31** for example can at least partially enter in the lower end of the rotor to which the processing container **5** belongs, and be fixedly connected for example with the fixed frame **27.**

Clearly, such a male-female coupling can also be inverted, fixing the centring pin **31** to the lower end of the rotor to which the processing container **5** belongs and forming the corresponding female seat on the fixed frame **27.**

The centring pin **31** can be made for example of steel or another metal, or of a suitable technopolymer.

Such a mounting system of the rotor is particularly simple, cost-effective, strong, longlasting and does not excessively rigidly constrain the structure.

The rotation axis ARR is constrained, by the bearing **29,** by the centring pin **31** or by other systems for suspending the processing container **5** and making it rotate on itself, so as to have an inclination preferably comprised between **0-30°,** and more preferably between **0-15°** with respect to the acceleration of local gravity.

The washer **1, 1'** can for example be provided with one or more nozzles (not shown) arranged to spray water, aqueous solutions, steam or other washing, sanitizing or sterilizing liquids on the stack of pallets P or other items to be washed.

Such nozzles can be arranged inside or outside of the processing container and be mounted on ramps or other systems of pipes, or even on self-propelled mechanical arms.

The washer **1, 1'** can also be provided with a drying system for drying the pallets or in any case for removing the residual water by blowing hot or cold air, or irradiating them with infrared or ultraviolet rays emitted by suitable lamps.

The washer **1, 1'** can be part of a more complex system indicated with the overall reference numeral **33,** and comprising for example one or more continuous conveyers **35, 36** that respectively carry pallets P or other items to be washed already stacked to the washer **1** and pick them up - advantageously already stacked - from such a washer **1, 1'** (Figure **1**).

Such continuous conveyers **35, 36** can for example be planes with idle or motorised rollers or conveyor belts.

An example of possible operation and use of the washer **1** described earlier is now described.

A stack of pallets P to be washed, sanitized or sterilized, for example europallets, is transported by the continuous conveyer **35** to the automatic washer **1** and is introduced in the cage or other processing container **5.**

The stack of pallets P is pushed against the rear wall of the processing container **5** until a side of the stack rests against the guide upright **15** and its brackets or forked hooks **13** are inserted in the spaces between the loading layers **39** of the stacked pallets P (Figures **3****,** 5A, 9) .

In particular, one or more feet **41** can be slotted in a fork formed by two or more lifting hooks or brackets **13** (Figure **9**).

The leaves **25** of the door or gate of the processing container **5** close, for example by rotating as shown by the arrows F**1**, F**2** and the locking brackets **210** fixed on the leaves **25** are introduced in the spaces between one pallet and the other, in particular in the spaces between the loading platforms of two adjacent pallets.

By closing, the leaves **25** also keep the stack in shape, preventing it from coming apart.

The assembly of the sliders **17** connected in a chain is lifted by their actuator **45'** lifting the pallets P on only one side, i.e. on only one side of the stack allowing the washing, sanitizing or sterilizing nozzles to easily and properly spray also the lower surface of the feet and of the strips **43** with water, steam, aqueous solutions, chemical detergents - for example containing surfactants and/or degreasing substances - or chemical disinfectants, while the locking brackets **210** located on the opposite side of the stack lock the pallets P preventing them from lifting (Figure **5**) .

Once the washing, sanitizing or sterilizing processing has ended, the stack of pallets can be dried by making the processing container **5** rotate on itself so as to remove the washing water or other processing liquid through centrifugal force.

In addition or alternatively, the stack of pallets P can be dried with a stream of hot air or by irradiating it with heating lamps, for example infrared.

After this the stack of pallets can be extracted from the processing container **5** and transported downstream for example through the continuous conveyers **36.**

The opening of the leaves **25** of an automatic washer according to the present invention can be carried out by hand by a human operator, or automatically by the washer machine itself through a suitable automatic door-opening system, like for example that of the embodiment of Figures **14-17****.**

Such an automatic door-opening system, arranged to reversibly and automatically open and close the leaves 25 or other door or doors of the processing container **5",** advantageously comprises one or more door-opening and closing actuators **51** arranged for example at or close to the roof **53** of the processing container **5".**

Such a roof **53** can be formed from a suitable lid **54,** for example disc-shaped (Figure **14**).

The door-opening and closing actuators **51** can comprise for example hydraulic or pneumatic cylinders, an end of which is fixed preferably at or close to the rotation axis AR of the processing container **5",** whereas the other end of each hydraulic or pneumatic cylinder **51** can be fixed to a leaf **25",** in an area thereof sufficiently far from the hinges that fix the leaf **25"** itself to the rest of the processing container **5",** so as to have a sufficient lever arm to open and close such a leaf **25".**

Fixing an end of the pneumatic or hydraulic cylinders **51** close to the rotation axis AR allows the cylinders **51** be fed more easily with hydraulic oil or compressed air through a suitable sealed rotating joint, *per se* known and mounted coaxial to the axis AR, feeding the hydraulic or pneumatic cylinders **51, 55** mounted on a rotating member with hydraulic oil or compressed air; one part of such a rotating joint is mounted on the fixed frame of the automatic washer **1, 1'** whereas another part is fixedly connected with the rotor formed by the processing container **5, 5', 5"** and rotates together with it.

Such an automatic door opening system advantageously comprises an automatic door-locking system arranged to reversibly lock and unlock the leaves **25** for example in their closed position.

Such an automatic door-locking system advantageously comprises one or more latches **53**A, **53**B and a latch actuator **55,** all preferably mounted on each leaf **25** (Figures 15, 15A).

Advantageously, each leaf **25** of the door - or doors - of the processing container **5"** has at least a lower latch **53**A and an upper latch **53**B mounted on it, the first arranged to engage with the lower threshold of the door and the second arranged to engage with the upper threshold of the door of the processing container **5"**.

The latches **53**A, **53**B can be or comprise bars for example metal, for example with circular section.

The free ends of the latches **53**A**, 53**B are arranged to engage in suitable holes or other female seats formed respectively in the base **9** and in the lid **54** of the processing container **5",** reversibly inserting in such holes so as to firmly lock the leaves **25** for example in their closed position (Figures 15, 15A).

Thus locked, the leaves **25** run less of a risk of accidentally opening for example if during the rotation of the processing container **5"** some pallet P rest against the leaves **25** themselves or collides with them, for example due to an imperfect balancing of the rotor formed by the container **5",** undesired vibrations or other accidents.

Each latch actuator **55** is arranged to actuate one or more latches **53**A**, 53**B for example rotating, or in any case moving, the main lever **57.**

Each latch actuator **55** can for example comprise a pneumatic or hydraulic cylinder (Figures **14-17**) or even a suitable electromechanical actuator, like for example a solenoid actuator.

The automatic door-locking system advantageously comprises a lever system that transmits the motion of each latch actuator **55** to one or more latches **53**A**, 53**B**.**

Such a lever system can comprise for example a main lever **57** and two connection bars **59.**

Each connection bar **59** is connected through suitable articulations, at one end to a respective main lever **57,** and at the other end thereof to a lower **53**A or upper latch **53**B (Figures **16****,** **17**).

Each main lever **57** can be fixed, through a suitable hinge **61,** to a upright of a leaf **25".**

Preferably, by moving ideally along the main lever **57,** the hinge **61** is arranged between the fixing points of two different connection bars **59** and one of the fixing points of one of said connection bars **59** is arranged between the fixing point on the lever **57** of the latch actuator **55** and the hinge **61,** giving the latch actuator **55** a greater lever arm.

Figure **16** shows the automatic locking system in unlocked position, with the latches **53**A**, 53**B extracted from the engagement holes in the base **9.**

Figures **15**A**, 17,** on the other hand, show the automatic locking system in locked position, with the latches **53**A**, 53**B inserted in the engagement holes in the base **9.**

The automatic door-opening and locking systems are advantageous for opening and closing the leaves **25, 25', 25"** of large automatic washers, the processing container **5, 5' 5"** of which can form an inner chamber even many metres high, they allow the leaves **25** be opened and closed keeping the staff away, thus reducing the risks of injury, and in general they make allow the opening, closing, locking and unlocking operations of the leaves **25** be carried out more quickly, thus increasing the productivity of the washer **1.**

An example of possible operation and use of the washer **1'** described earlier is now described.

A stack of pallets P or other substantially flat items to be washed is introduced in the processing container **5'**, for example transported by the upstream continuous conveyer **35** (Figure **13A****).**

As stated, the processing container **5'** can be fixed with respect to the fixed frame **27** of the washer **1',** but in other embodiments that are not shown it can be mounted on a rotor so as to be able to rotate on itself, for example around a vertical rotation axis and centrifuge its load.

While the stack is introduced in the processing container **5'** the lifting columns **15'.1, 15'.2, 15'.3, 15' .4** are in non-operating position, rotated so that the lifting brackets **13'** do not project towards the inside of the space of the container **5'** in which the washing or other processing takes place; more in particular the lifting brackets **13'** are substantially longitudinal to the entry and/or exit direction of the stack into/from the processing container **5'**.

The possible leaves **25, 25', 25"** or other doors of the processing container **5'** close, the photocell or other detection system of the height determines the height H**1,** H**2** of the single pallets and communicates it to the PLC or other logic control unit of the washer **1'** which, based on such a detection actuates the most suitable pair of lifting columns **15'**, for example the columns **15'.1** and **15'.4** of Figure **10** close to a diagonal of the plan of the pallets P, switching them to the operating condition and thus inserting the lifting brackets **13'** of these two columns in the spaces between two adjacent pallets of the stack.

The actuator **45'** for example of the column **15'.1** can be actuated so as to keep the relative sliders **17** as low as possible or in any case to prevent them from lifting, thus preventing the relative brackets **13'** and the edges of the pallets P from lifting on the adjacent side of the stack.

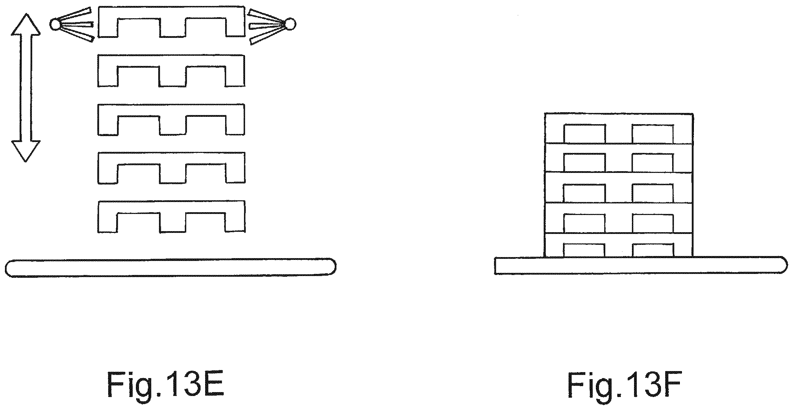

The actuator **45'** for example of the column **15'.4** can, on the other hand, be actuated so as to lift the chain of the relative sliders **17** thus lifting the pallets P only on the side of the stack adjacent to the column **15'.4** itself, and thus tilting them all or almost all towards the opposite side of the stack (Figure **13**C**).**

Suitable washing, sanitizing or sterilizing nozzles not shown thus spray water, steam, aqueous solutions, detergents or chemical disinfectants both on the upper and lower surface of the feet **41** and of the strips **43** on the side of the columns **15'.3, 15'.4,** after which the actuator **45'** of the column **15'.4** is again actuated so as to lower the chain of the relative sliders **17** and the relative brackets **13'** laying down the pallets P, which are stacked again with the loading layers **39** substantially horizontal.

The actuator **45'** for example of the column **15'.4** can then be actuated so as to keep the relative sliders **17** as low as possible or in any case prevent them from lifting, thus preventing the relative brackets **13'** and the edges of the pallets P from lifting on the adjacent side of the stack, while the actuator **45'** for example of the column **15'.1** can, on the other hand, be actuated so as to lift the chain of the relative sliders **17** thus lifting the pallets P only on the side of the stack adjacent to the column **15'.1** itself, and thus tilting all or almost all of them towards the opposite side of the stack (Figure 13D).

The aforementioned washing, sanitizing or sterilizing nozzles, not shown, thus spray further water, steam, aqueous solutions, detergents or chemical disinfectants both on the upper surface and on that of the feet **41** and of the strips **43** on the side of the columns **15'.1, 15'.2,** after which the actuator **45'** of the column **15'.1** is actuated so as to lower the chain of the relative sliders **17** and the relative brackets **13'** laying down the pallets P, which are stacked again with the loading layers **39** substantially horizontal (Figure 13F).

Possibly, between the liftings of Figures 13A and **13**C and/or between the liftings of Figures 13D and 13F the lifting columns **15'.1** and **15'.4** can be actuated so as to lift both of the side edges of one or more pallets P without tilting them on one side, but on the other hand keeping the pallets substantially horizontal (Figures **13**B**, 13**E).

The washer **1'** can thus possibly dry the stack of pallets P for example with a stream of hot air or with infrared ray lamps, after which all of the columns **15'.1, 15'.2, 15'.3, 15'.4** are placed in the non-operating condition, for example rotated so as to orient the brackets **13'** according to the entry and/or exit direction of the stacks of pallets into or from the processing chamber; the possible doors of the processing container **5'** open and the possible continuous conveyer **36** extracts the stack of washed, sanitized or sterilized pallets P and sends them downstream.

From the above description the advantages of the washers **1, 1'** according to the invention are clear to see: they allow substantially the whole surface of the pallets P or other items to be processed be very deeply and accurately washed, sanitized or sterilized, including their lower surface, even if they are introduced in the processing container **5, 5'** stacked and extracted from it still stacked.

Since it is necessary neither to disassemble the stacks of pallets before introducing them in the washer **1, 1'**, nor to assemble the stacks again after the pallets have been extracted from it, the movement of the pallets in the factory where it is used can be substantially simplified, sped up and automated, facilitating in particular the automatic movement of the stacks of pallets with continuous conveyers, such as roller planes or conveyor belts, or even with simple fork lifts: the pallets P can indeed be loaded stacked in to and unloaded still stacked from the processing container **5, 5', 5"** by simply forking them.

The greater efficiency and speed of washing or more generally of spraying of the washers **1, 1'** also derives from the fact that when the various pallets of a stack are all spaced and tilted on one side, the jets of the nozzles transversally and simultaneously hit a larger portion both of the larger upper face and of the larger lower face of the various pallets (Figures 13C, 13D) .

Inclining the stacked pallets by lifting them and spacing them only on one side, and simultaneously rotating the stack with respect to the nozzles that spray it with water or other washing fluid makes the washing much more effective, for example with respect to the simple lifting and spacing of the pallets only on one side without rotating the stack; for example the rotation of the stack simultaneously with the lifting and tilting on one side allows the water - or other washing liquid - to better penetrate in the most hidden recesses of the stack and of the pallets, and to them flow away more easily from them, for example thanks to the centrifugal effect.

The washers **1, 1'** are particularly suitable for operating as pass-through working stations, which are simply crossed by the flow of pallets or other processed items: the dirty pallets can easily enter on one side of the washer **1, 1'** and be extracted from the opposite side.

The washers **1, 1'** are also particularly suitable for processing pallets P and other substantially flat items of different shapes and sizes with high production rates, as well as suitable for being made as washers capable of drying the stacked processed items through centrifuging or without centrifuging.

The author of the present invention has managed to construct for example washers **1, 1'** capable of carrying out **8-10** washing cycles per hour of pallets for agrobusiness and for large-scale distribution.

For example, the present invention can be applied to make not only washers but also automatic machines that sanitize, sterilize, paint, make surface finishes or carry out yet other treatments on pallets or other substantially flat items to be processed P.

The lifting system of a washer according to the invention can also be provided with two, three, five and more guide uprights provided with respective sliders and lifting hooks or brackets, so as to be able to tilt the stacked pallets by gripping them simultaneously on a single side with the lifting brackets of two or more guide uprights and not of a single guide upright like in figures **5****,** **7****.**

The brackets **13, 13'**, hooks, clamps, supports or pushers that tilt the pallets P or other items to be processed by lifting an outer edge thereof can be moved for example by one or more bars or by a continuous conveyer for example with a chain instead of sliding on a respective guide upright (**15, 15'**).

Any reference in this description to "an embodiment", "an example of embodiment" means that a particular feature or structure described in relation to such an embodiment is comprised in at least one embodiment of the invention and in particular in a particular variant of the invention as defined in a main claim.

The fact that such expressions appear in various passages of the description does not imply that they necessarily refer only to the same embodiment.

Moreover, when a feature, element or structure is described in relation to a particular embodiment, it should be observed that it is within the capabilities of those skilled in the art to apply such a feature, element or structure to other embodiments.

Reference numerals that differ only for different superscripts, e.g. **21', 21", 21**^{III} unless specified otherwise indicate different variants of an element called in the same way.

Moreover, all of the details can be replaced by technically equivalent elements.

For example, the materials used, as well as the sizes, can be whatever according to the technical requirements.

It should be understood that an expression of the type "A *comprises* B, C, D" or "A *is formed from* B, C, D" also comprises and describes the particular case in which "A *consists of* B, C, D".

The expression *"A comprises an element B"* unless specified otherwise should be interpreted as *"A comprises one or more elements B".*

The examples and lists of possible variants of the present application should be taken as nonexhaustive lists.

## Claims

1. Processing device (**1, 1'**) for processing substantially flat items to be processed such as pallets (P), platforms, pans, boxes, crates or plates for example by washing, sanitizing or sterilizing them, comprising:
- a processing container (**5, 5'**) arranged for containing a plurality of items to be processed (P) stacked one on top of the other;
- a lifting system **(11,11')** arranged for lifting at least part of the items to be processed (P) tilting and spacing them while they are stacked inside the processing container (**5, 5'**);
- one or more nozzles arranged for spraying the items to be processed (P) stacked in the processing container (**5, 5'**) with a work fluid such as for example water, an aqueous solution, steam, a chemical detergent or a disinfectant, spraying them for example while such items to be processed are tilted and stacked;
**characterized by** further comprising a rotor on which the processing container (**5, 5'**) is mounted and which is arranged for rotating around itself, together with the processing container (**5, 5'**), around a substantially vertical rotation axis (ARR) so as to accelerate the drying of the washed items to be processed (P) by centrifuging them.

2. Processing device (**1, 1'**) according to claim **1,** wherein the lifting system **(11,11')** is arranged for lifting at least part of the items to be processed (P) inside the processing container (**5, 5'**) tilting them all on the same side.

3. Processing device (**1, 1'**) according to claim **1** or **2,** wherein the lifting system (**11, 11'**) comprises one or more brackets (**13, 13'**), hooks, clamps or pushers arranged for tilting one or more items to be processed (P) pushing or pulling at or near their outer edge.

4. Processing device (**1, 1'**) according to claim **3,** wherein the lifting system (**11, 11'**) comprises one or more lifting columns **(15'.1, 15'2, 15'.3, 15'.4)** each of which in turn comprises a guide upright (**15, 15'**) and one or more sliders (**17, 17'**), and wherein:
- one or more of said brackets (**13, 13'**), hooks, clamps or pushers are fastened to each slider (**17, 17'**);
- the one or more sliders (**17, 17'**) sliding along the respective guide upright (**15, 15'**) of the lifting column are arranged for tilting the one or more items to be processed (P) pushing or pulling them through the respective brackets (**13, 13'**), hooks, clamps or pushers.

5. Processing device (**1, 1'**) according to claim **4,** wherein a plurality of sliders (**17, 17'**) of at least one and the same lifting column are connected together in order to substantially form a chain, rope or other articulated assembly.

6. Processing device (**1, 1'**) according to one or more of the previous claims, arranged for lifting, by tilting, at least part of the items to be processed (P) stacked inside the processing container (**5, 5'**) pushing or pulling them at or near two different sides of the stack formed by the items to be processed (P).

7. Processing device (**1, 1'**) according to claims **4** and **6,** comprising at least a first and a second lifting column, wherein the first lifting column is arranged for extending along a side of the stack of items to be processed (P) stacked inside the processing container (**5, 5')** other than the side of the stack along which the second lifting column extends, for example extending along a side of the stack opposite to that along which the second lifting column extends.

8. Processing device (**1, 1'**) according to at least claim **4,** in which the one or more lifting columns are arranged for switching reversibly from an operating condition in which the respective brackets (**13, 13'**), hooks, clamps or pushers can engage with the items to be processed (P) stacked inside the processing container (**5, 5'**) sliding along the respective guide upright (**15, 15'**), to an non-operating condition in which the respective brackets (**13, 13'**), hooks, clamps or pushers cannot engage with the items to be processed (P) stacked inside the processing container (**5, 5'**) sliding along the respective guide upright (**15, 15'**).

9. Processing device (**1, 1'**) according to claim **8,** wherein the one or more lifting columns are arranged for switching reversibly from the operating condition to the non-operating condition with one or more of the following movements: rotating around itself and around its own longitudinal axis, rotating, translating, rotating and translating with respect to the rest of the processing device (**1, 1'**).

10. Processing device **(1, 1')** according to at least claim **4,** comprising:
- at least a first lifting column arranged for lifting and tilting one stack of first items to be processed;
- at least a second lifting column arranged for lifting and tilting one stack of second items to be processed, where the height (H**1**), the width (W**1**) or the length (L**1**) of the first items to be processed are different from the height (H**2**), the width (W**2**) or the length (L**2**) of the second items to be processed;
- a detection system;
and the processing device **(1, 1'**) is programmed or in any case arranged for carrying out automatically the following operations:
- detecting by the detection system at least one of the height (H**1**, H**2**), the width (W**1**, W**2**) and the length (L**1**, L**2**) of the items to be processed introduced in a stacked condition in the processing container **(5, 5');**
- based on the detections of the detection system, setting the at least one first or the at least one second lifting column in an operating condition so as to be able, using the latter, to lift and tilt the items to be processed introduced in a stacked condition in the processing container **(5, 5').**

11. Method for processing substantially flat items to be processed pans, boxes, crates or plates for example by washing, sanitizing or sterilizing them , comprising the following operations:
- providing a processing device **(1, 1')** having the features as those of one or more of the previous claims;
- introducing a stack of items to be processed (P) in the processing container (**5, 5'**);
- cause the rotor to rotate around itself, together with the processing container (**5, 5'**), around a substantially vertical rotation axis (ARR) so as to accelerate the drying of the washed items to be processed (P) by centrifuging them.
- tilting on one and the same side one or more items to be processed (P) keeping them resting on their stack, lifting them on one side using the one or more brackets (**13, 13'**), hooks, clamps or pushers.

12. Method according to claim **11,** comprising the operation of spraying with an operating fluid the lower face of at least one of the items to be processed (P), for example by washing, sanitizing or sterilizing it, at least while such item to be processed (P) is tilted on one side and resting on the stack formed by the other items to be processed (P).

## Patentansprüche

1. Bearbeitungsvorrichtung (**1, 1'**) zur Bearbeitung von im Wesentlichen flachen, zu bearbeitenden Gegenständen, wie Paletten (P), Plattformen, Pfannen, Schachteln, Kisten oder Platten, zum Beispiel durch Waschen, Desinfizieren oder Sterilisieren derselben, umfassend:
- einen Bearbeitungsbehälter **(5, 5'),** der so angeordnet ist, dass er eine Vielzahl von übereinander gestapelten, zu bearbeitenden Gegenständen (P) enthält;
- ein Hebesystem **(11, 11'),** das zum Anheben mindestens eines Teils der zu bearbeitenden Gegenstände (P), zum Kippen und zum Beabstanden derselben angeordnet ist, während sie im Inneren des Bearbeitungsbehälters **(5, 5')** gestapelt sind;
- eine oder mehrere Düsen, die so angeordnet sind, dass sie die in dem Bearbeitungsbehälter **(5, 5')** gestapelten, zu bearbeitenden Gegenstände (P) mit einer Arbeitsflüssigkeit, wie z. B. Wasser, einer wässrigen Lösung, Dampf, einem chemischen Reinigungsmittel oder einem Desinfektionsmittel, besprühen, z. B. während die zu bearbeitenden Gegenstände gekippt und gestapelt sind;
**dadurch gekennzeichnet, dass** sie ferner einen Rotor umfasst, auf dem der Bearbeitungsbehälter **(5, 5')** montiert ist und der so angeordnet ist, dass er sich zusammen mit dem Bearbeitungsbehälter **(5, 5')** um eine im Wesentlichen vertikale Drehachse (ARR) dreht, um die Trocknung der zu bearbeitenden, gewaschenen Gegenstände (P) durch Zentrifugieren derselben zu beschleunigen.

2. Bearbeitungsvorrichtung **(1, 1')** nach Anspruch **1,** wobei das Hebesystem **(11, 11')** so angeordnet ist, dass es mindestens einen Teil der zu bearbeitenden Gegenstände (P) innerhalb des Bearbeitungsbehälters **(5, 5')** anhebt und sie alle auf dieselbe Seite kippt.

3. Bearbeitungsvorrichtung **(1, 1')** nach Anspruch **1 oder 2,** wobei das Hebesystem **(11, 11')** einen oder mehrere Bügel **(13, 13'),** Haken, Klammern oder Schubelemente umfasst, die zum Kippen eines oder mehrerer zu bearbeitender Gegenstände (P) angeordnet sind, indem sie sie an oder nahe ihrer Außenkante schieben oder ziehen.

4. Bearbeitungsvorrichtung **(1, 1')** nach Anspruch **3,** wobei das Hebesystem **(11, 11')** eine oder mehrere Hebesäulen **(15'.1, 15'2, 15'.3, 15'.4)** umfasst, von denen jede ihrerseits eine Führungssäule **(15, 15')** und einen oder mehrere Schieber **(17, 17')** umfasst, und wobei:
- einer oder mehrere der Bügel **(13, 13'),** Haken, Klammern oder Schubelemente an jedem Schieber **(17, 17')** befestigt sind;
- der eine oder die mehreren Schieber **(17, 17'),** die entlang der jeweiligen Führungssäule **(15, 15')** der Hebesäule gleiten, sind so angeordnet, dass sie den einen oder die mehreren zu bearbeitenden Gegenstände (P) kippen und sie durch die jeweiligen Bügel **(13, 13'),** Haken, Klammern oder Schubelemente schieben oder ziehen.

5. Bearbeitungsvorrichtung **(1, 1')** nach Anspruch **4,** wobei eine Vielzahl von Schiebern **(17, 17')** mindestens einer und derselben Hebesäule miteinander verbunden sind, um im Wesentlichen eine Kette, ein Seil oder eine andere gelenkige Anordnung zu bilden.

6. Bearbeitungsvorrichtung **(1, 1')** nach einem oder mehreren der vorstehenden Ansprüche, die so angeordnet ist, dass sie mindestens einen Teil der zu bearbeitenden Artikel (P), die im Bearbeitungsbehälter **(5, 5')** gestapelt sind, durch Kippen anhebt, indem sie sie an oder nahe von zwei verschiedenen Seiten des von den zu bearbeitenden Gegenständen (P) gebildeten Stapels schiebt oder zieht.

7. Bearbeitungsvorrichtung **(1, 1')** nach den Ansprüchen **4** und **6,** umfassend mindestens eine erste und eine zweite Hebesäule, wobei die erste Hebesäule so angeordnet ist, dass sie sich entlang einer anderen Seite des Stapels von zu bearbeitenden Gegenständen (P) erstreckt, die innerhalb des Bearbeitungsbehälters **(5, 5')** gestapelt sind, als die Seite des Stapels, entlang der sich die zweite Hebesäule erstreckt, zum Beispiel entlang einer Seite des Stapels, die derjenigen gegenüberliegt, entlang der sich die zweite Hebesäule erstreckt.

8. Bearbeitungsvorrichtung **(1, 1')** nach mindestens Anspruch **4,** wobei die eine oder mehreren Hebesäulen so angeordnet sind, dass sie reversibel von einem Betriebszustand, in dem die jeweiligen Bügel **(13, 13'),** Haken, Klammern oder Schubelemente mit den im Inneren des Bearbeitungsbehälters **(5, 5')** gestapelten, zu bearbeitenden Gegenständen (P) in Eingriff kommen können, die entlang der jeweiligen Führungssäule **(15, 15')** gleiten, in einen Nichtbetriebszustand umschalten können, in dem die jeweiligen Bügel **(13, 13'),** Haken, Klammern oder Schubelemente nicht mit den im Inneren des Bearbeitungsbehälters **(5, 5')** gestapelten, zu bearbeitenden Gegenständen (P) in Eingriff kommen können, die entlang der jeweiligen Führung **(15, 15')** gleiten.

9. Bearbeitungsvorrichtung **(1, 1')** nach Anspruch **8,**
wobei die eine oder die mehreren Hebesäulen angeordnet sind, um mit einer oder mehreren der folgenden Bewegungen reversibel vom Betriebszustand in den Nichtbetriebszustand umzuschalten: Drehen um sich selbst und um die eigene Längsachse, Drehen, Verschieben, Drehen und Verschieben in Bezug auf den Rest der Bearbeitungsvorrichtung **(1, 1')**.

10. Bearbeitungsvorrichtung **(1, 1')** nach mindestens Anspruch **4,** umfassend:
- mindestens eine erste Hebesäule, die zum Anheben und Kippen eines Stapels von ersten zu bearbeitenden Gegenständen angeordnet ist;
- mindestens eine zweite Hebesäule, die zum Anheben und Kippen eines Stapels von zweiten zu bearbeitenden Gegenständen angeordnet ist, wobei die Höhe **(HI),** die Breite **(WI)** oder die Länge **(LI)** der ersten zu bearbeitenden Gegenstände von der Höhe **(H2),** der Breite **(W2)** oder der Länge **(L2)**
der zweiten zu bearbeitenden Gegenstände abweichen;
- ein Erfassungssystem;
und die Bearbeitungsvorrichtung **(1, 1')** ist so programmiert oder auf jeden Fall angeordnet, dass sie die folgenden Vorgänge automatisch ausführt:
- Erfassen, durch das Erfassungssystem, von mindestens einem von der Höhe **(HI, H2),** der Breite **(WI, W2)** und der Länge **(LI, L2)** der zu bearbeitenden Gegenstände, die in gestapeltem Zustand in den Bearbeitungsbehälter **(5, 5')** eingeführt werden;
- auf der Grundlage der Erfassungen des Erfassungssystems,
Versetzen der mindestens einen ersten oder der mindestens einen zweiten Hebesäule in einen Betriebszustand, um unter Verwendung letzterer die in gestapeltem Zustand in den Bearbeitungsbehälter **(5, 5')** eingeführten, zu bearbeitenden Gegenstände anheben und kippen zu können.

11. Verfahren zur Bearbeitung von im Wesentlichen flachen Gegenständen, wie z. B. Pfannen, Schachteln, Kisten oder Platten, durch Waschen, Desinfizieren oder Sterilisieren derselben, umfassend die folgenden Schritte:
- Bereitstellen einer Bearbeitungsvorrichtung **(1, 1')** mit den Merkmalen eines oder mehrerer der vorstehenden Ansprüche;
- Einführen eines Stapels von zu bearbeitenden Gegenständen (P) in den Bearbeitungsbehälter **(5, 5');**
- in Drehung Versetzen des Rotors zusammen mit dem Bearbeitungsbehälter **(5, 5')** um eine im Wesentlichen vertikale Drehachse (ARR) um sich selbst, um die Trocknung der gewaschenen und zu bearbeitenden Teile (P) durch Zentrifugieren derselben zu beschleunigen.
- Kippen eines oder mehrerer zu bearbeitender Gegenstände (P) auf ein und dieselbe Seite, wobei sie auf ihrem Stapel liegen bleiben, Anheben derselben auf einer Seite unter Verwendung des einen oder der mehreren Bügel **(13, 13'),** Haken, Klammern oder Schubelemente.

12. Verfahren nach Anspruch **11,** umfassend den Vorgang des Besprühens der Unterseite mindestens eines der zu bearbeitenden Gegenstände (P) mit einer Betriebsflüssigkeit, zum Beispiel durch Waschen, Desinfizieren oder Sterilisieren, mindestens während ein solcher zu bearbeitender Gegenstand (P) auf eine Seite gekippt wird und auf dem von den anderen zu bearbeitenden Gegenständen (P) gebildeten Stapel ruht.

## Revendications

1. Dispositif de traitement (**1, 1'**) pour le traitement d'articles sensiblement plats à traiter tels que des palettes (P), des plates-formes, des poêles, des boites, des caisses ou des assiettes, par exemple en les lavant, en les désinfectant ou en les stérilisant, comprenant:
- un récipient de traitement **(5, 5')** conçu pour contenir une pluralité d'articles à traiter (P) empilés les uns sur les autres;
- un système de levage (**11, 11'**) conçu pour soulever au moins une partie des articles à traiter (P), les incliner et les espacer pendant qu'ils sont empilés à l'intérieur du récipient de traitement **(5, 5');**
- une ou plusieurs buses conçues pour pulvériser les articles à traiter (P) empilés dans le récipient de traitement **(5,5')** avec un fluide de travail tel que, par exemple, de l'eau, une solution aqueuse, de la vapeur, un détergent chimique ou un désinfectant, en les pulvérisant par exemple lorsque les articles à traiter sont inclinés et empilés;
**caractérisé en ce qu'il** comprend en outre un rotor sur lequel est monté le récipient de traitement **(5, 5')** et qui est conçu pour tourner sur lui-même, avec le récipient de traitement **(5, 5'),** autour d'un axe de rotation sensiblement vertical (ARR) afin d'accélérer le séchage des articles lavés à traiter (P) en les centrifugeant.

2. Dispositif de traitement **(1**, **1')** selon la revendication **1,** dans lequel le système de levage **(11, 11')** est conçu pour soulever au moins une partie des articles à traiter (P) à l'intérieur du récipient de traitement **(5, 5')** en les inclinant tous du même côté.

3. Dispositif de traitement **(1, 1')** selon la revendication **1 ou 2,** dans lequel le système de levage **(11, 11')** comprend un ou plusieurs supports **(13, 13'),** des crochets, des pinces ou des poussoirs disposés pour incliner un ou plusieurs articles à traiter (P) en les poussant ou en les tirant à leur bord extérieur ou à proximité de celui-ci.

4. Dispositif de traitement **(1, 1')** selon la revendication **3,** dans lequel le système de levage **(11, 11')** comprend une ou plusieurs colonnes de levage **(15'.1, 15'2, 15'.3, 15'.4)** dont chacune comprend à son tour un montant de guidage **(15, 15')** et une ou plusieurs glissières **(17, 17'),** et dans lequel:
- un ou plusieurs desdits supports **(13, 13'),** crochets, pinces ou poussoirs sont fixés à chaque glissière **(17, 17');**
- le ou les glissières **(17, 17')** coulissant le long du montant de guidage respectif **(15, 15')** de la colonne de levage sont conçues pour incliner le ou les articles à traiter (P) en les poussant ou en les tirant à travers les supports respectifs **(13, 13'),** les crochets, les pinces ou les poussoirs.

5. Dispositif de traitement **(1, 1')** selon la revendication **4,** dans lequel plusieurs glissières **(17, 17')** d'au moins une seule et même colonne de levage sont reliées entre elles afin de former sensiblement une chaîne, un câble ou un autre assemblage articulé.

6. Dispositif de traitement **(1, 1')** selon l'une ou plusieurs des revendications précédentes, conçu pour soulever, par inclinaison, au moins une partie des articles à traiter (P) empilés à l'intérieur du récipient de traitement **(5, 5')** en les poussant ou en les tirant sur ou à proximité de deux côtés différents de la pile formée par les articles à traiter (P).

7. Dispositif de traitement **(1, 1')** selon les revendications **4** et **6,** comprenant au moins une première et une seconde colonne de levage, dans laquelle la première colonne de levage est conçue pour s'étendre le long d'un côté de la pile d'articles à traiter (P) empilés à l'intérieur du récipient de traitement **(5, 5')** autre que le côté de la pile le long duquel s'étend la seconde colonne de levage, par exemple en s'étendant le long d'un côté de la pile opposé à celui le long duquel s'étend la seconde colonne de levage.

8. Dispositif de traitement **(1, 1')** selon au moins la revendication **4,** dans lequel la ou les colonnes de levage sont disposées pour passer de manière réversible d'un état de fonctionnement dans lequel les supports respectifs **(13, 13'),** crochets, pinces ou poussoirs peuvent s'engager avec les articles à traiter (P) empilés à l'intérieur du récipient de traitement **(5, 5')** coulissant le long du montant de guidage respectif **(15, 15'),** à un état de non fonctionnement dans lequel les supports **(13, 13'),** crochets, pinces ou poussoirs respectifs ne peuvent pas s'engager avec les articles à traiter (P) empilés à l'intérieur du récipient de traitement **(5, 5')** coulissant le long du montant de guidage respectif **(15, 15')**

9. Dispositif de traitement **(1, 1')** selon la revendication **8,**
dans lequel la ou les colonnes de levage sont conçues pour passer de manière réversible de l'état de fonctionnement à l'état de non fonctionnement avec un ou plusieurs des mouvements suivants : rotation sur lui-même et autour de son propre axe longitudinal, rotation, translation, rotation et translation par rapport au reste du dispositif de traitement **(1, 1').**

10. Dispositif de traitement **(1, 1')** selon au moins la revendication **4,** comprenant:
- au moins une première colonne de levage conçue pour soulever et incliner une pile de premiers articles à traiter;
- au moins une seconde colonne de levage conçue pour soulever et incliner une pile de seconds articles à traiter, lorsque la hauteur **(Hl)**, la largeur **(Wl)** ou la longueur **(LI)** des premiers articles à traiter sont différentes de la hauteur **(H2)*,*** la largeur **(W2)** ou la longueur **(L2)** des
seconds articles à traiter;
- un système de détection;
et le dispositif de traitement **(1, 1')** est programmé ou en tout cas conçu pour effectuer automatiquement les opérations suivantes:
- la détection par le système de détection d'au moins une des valeurs suivantes : la hauteur **(HI, H2),** la largeur **(WI, W2)** et la longueur **(LI, L2)** des articles à traiter introduits en pile dans le récipient de traitement **(5, 5');**
- sur la base des détections du système de détection,
la mise de l'au moins une première colonne de levage ou de l'au moins une seconde colonne de levage en état de fonctionnement afin de pouvoir, à l'aide de ces dernières, soulever et incliner les articles à traiter introduits en pile dans le récipient de traitement **(5, 5')**

11. Procédé de traitement d'éléments à traiter sensiblement plats, poêles, boites, caisses ou assiettes, par exemple en les lavant, en les désinfectant ou en les stérilisant, comprenant les opérations suivantes:
- la fourniture d'un dispositif de traitement **(1, 1')** ayant les caractéristiques d'une ou plusieurs des revendications précédentes;
- l'introduction d'une pile d'articles à traiter (P) dans le récipient de traitement **(5, 5');**
- la rotation du rotor sur lui-même, ainsi que le récipient de traitement **(5, 5'),** autour d'un axe de rotation sensiblement vertical (ARR) afin d'accélérer le séchage des articles lavés à traiter (P) par centrifugation.
- l'inclinaison d'un seul et même côté d'un ou de plusieurs articles à traiter (P) en les maintenant en appui sur leur pile, les soulever d'un côté à l'aide d'un ou plusieurs supports **(13, 13'),** crochets, pinces ou poussoirs.

12. Procédé selon la revendication **11,** comprenant l'opération de pulvérisation d'un fluide de fonctionnement sur la face inférieure d'au moins un des articles à traiter (P), par exemple en le lavant, le désinfectant ou le stérilisant, au moins pendant que cet article à traiter (P) est incliné d'un côté et repose sur la pile formée par les autres articles à traiter (P).
